# EUROPÄISCHE PATENTANMELDUNG

(11) **EP 1 997 819 A1**
(43) Veröffentlichungstag der Anmeldung: **03.12.2008**
(21) Anmeldenummer: 07108915.5
(22) Anmeldetag: 25.05.2007
(51) Int. Cl.: C07D 451/10

(54) **Verfahren zur Herstellung von Scopinestern**

(71) Anmelder: Boehringer Ingelheim Pharma GmbH & Co. KG, 55216 Ingelheim am Rhein (DE)
(72) Erfinder: Pfrengle, Waldemar, 88400, Biberach (DE); Rall, Werner, 88441, Mittelbiberach (DE); Brandenburg, Jörg, 65199, Wiesbaden (DE); Herter, Rolf, 88400, Biberach (DE)
(74) Vertreter: Hammann, Heinz

(57) **Zusammenfassung**

Die Erfindung betrifft ein neues Verfahren zur Herstellung von Scopinestern der allgemeinen Formel 1 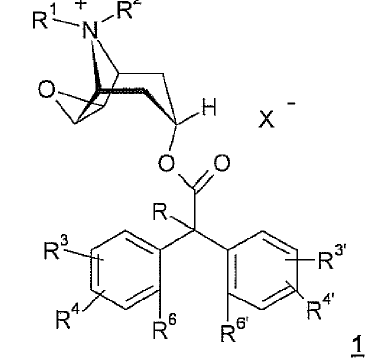 worin X⁻ R, R¹, R², R³, R⁴, R^{3'} ,R^{4'}, R⁶ und R^{6'} die in den Ansprüchen und in der Beschreibung genannten Bedeutungen haben können.

## Beschreibung

Die Erfindung betrifft ein neues Verfahren zur Herstellung von Scopinestern der allgemeinen Formel **1** 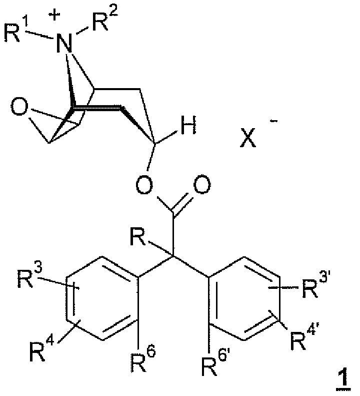 worin X - und die Gruppen R, R¹, R², R³, R^{3'}, R⁴ und R^{4'}, R⁶ und R^{6'} die in den Ansprüchen und in der Beschreibung genannten Bedeutungen haben können.

### Hintergrund der Erfindung

Anticholinergika können bei einer Vielzahl von Erkrankungen therapeutisch sinnvoll eingesetzt werden. Hervorzuheben sind hier beispielsweise die Therapie von Asthma oder COPD (chronic obstructive pulmonary disease = chronisch obstruktive Lungenerkrankung). Zur Therapie dieser Erkrankungen werden beispielsweise durch die WO 92/16528 Anticholinergika vorgeschlagen, die ein Scopin-, Tropenol- oder auch Tropin-Grundgerüst aufweisen. In WO 92/16528 werden auch Verfahren zur Herstellung dieser Anticholinergika erwähnt. Weitere Verfahren zur Herstellung von Estern des Scopins werden in EP 418 716 A1 offenbart.

Die Scopinester der allgemeinen Formel **1** sind bereits aus der WO 031964419 A1 bekannt. Weiterhin offenbart auch WO 03/064419 A1 Verfahren zur Herstellung der Scopinester der allgemeinen Formel **1**.

Neben den in der WO 92/16528 offenbarten Syntheseverfahren zur Herstellung von Scopinestern werden beispielsweise auch in der EP 418 716 A1 Verfahren zur Herstellung von Estern des Scopins offenbart. Dieaus WO 92/16528 und aus EP 418 716 A1 sowie aus der WO 92/16528 bekannten Herstellverfahren sind auch zur Herstellung der Verbindungen der Formel **1** anwendbar. Allerdings handelt es sich bei diesen synthetischen Zugängen um teilweise sehr komplexe, aus mehreren Syntheseschritten bestehende Vorgehensweisen.

Aufgabe der vorliegenden Erfindung ist es daher, ein Syntheseverfahren zur Herstellung der Scopinester nach Formel **1** bereitzustellen, welches einen einfacheren synthetischen Zugang zu den Verbindungen der allgemeinen Formel **1** ermöglicht, vorzugsweise ein einstufiges Syntheseverfahren.

### Detaillierte Beschreibung der Erfindung

Überraschenderweise wurde gefunden, daß Verbindungen der Formel **1** 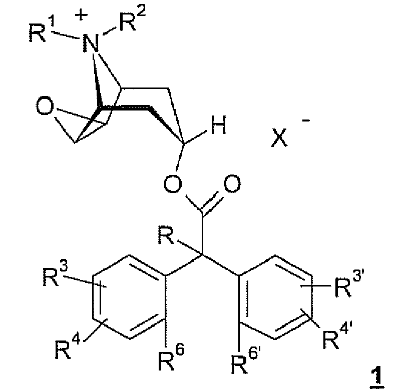 worin X - und die Gruppen R, R¹, R², R³, R^{3'}, R⁴, R^{4'}, R⁶ und R^{6'} die nachstehend genannten Bedeutungen haben, in einem einzigen Reaktionsschritt erhalten werden können, wenn als Ausgangsmaterial Verbindungen der Formel 2 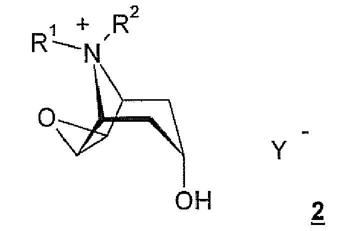 zum Einsatz gelangen.

Dementsprechend betrifft die vorliegende Erfindung ein Verfahren zur Herstellung von Verbindungen der Formel **1** 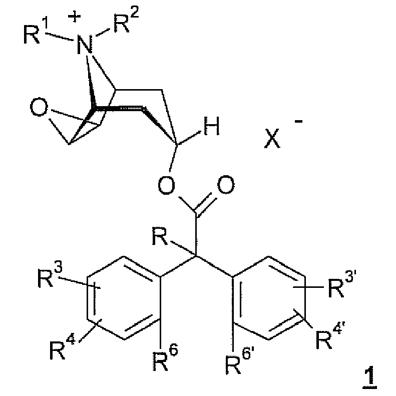 worin
- X⁻: ein einfach negativ geladenes Anion, vorzugsweise ein Anion ausgewählt aus der Gruppe bestehend aus Chlor, Brom, Iod, Methansulfonat oder Trifluormethansulfonat;
- R: Wasserstoff, Hydroxy, Methyl, Ethyl, -CF₃, CHF₂ oder Fluor;
- R¹ und R²: gleich oder verschieden, -C₁-C₅-Alkyl, welches gegebenenfalls durch -C₃-C₆-Cycloalkyl, Hydroxy oder Halogen substituiert sein kann,
oder
R¹ und R² gemeinsam eine -C₃-C₅-Alkylen-Brücke;
- R³, R⁴, R^{3'} und R^{4'},: gleich oder verschieden, Wasserstoff, -C₁-C₄-Alkyl, -C₁-C₄-Alkyloxy, Hydroxy, -CF₃, -CHF₂, CN, NO₂ oder Halogen,
- R⁶ und R^{6'}: Wasserstoff oder gemeinsam eine Einfachbindung bedeuten,
dadurch gekennzeichnet, daß eine Verbindung der Formel 2 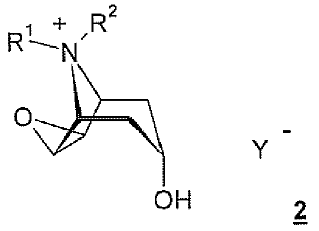 worin
- Y -: ein einfach negativ geladenes Anion, vorzugsweise Chlor, Brom, Iod, Methansulfonat oder Trifluormethansulfonat bedeuten kann, und
- R¹ und R²: die oben genannten Bedeutungen haben kann,
in einem Schritt mit einer Verbindung der Formel **3** 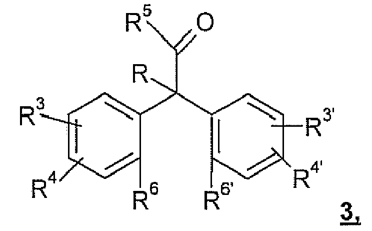 worin
- R⁵: ein Rest ausgewählt aus der Gruppe bestehend aus Hydroxy, Methoxy, Ethoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet und
worin R, R3, R^{3'}, R⁴ , R^{4'} , R⁶ und R^{6'} die vorstehend genannten Bedeutungen haben, umgesetzt wird.

Bevorzugt betrifft die vorliegende Erfindung ein Verfahren zur Herstellung von Verbindungen der Formel **1** 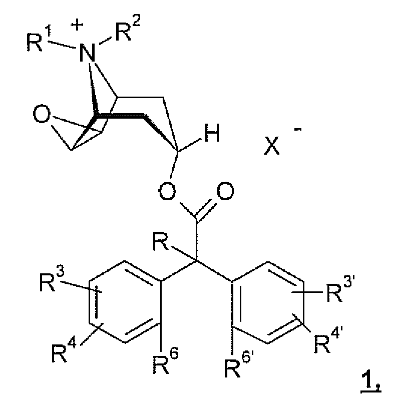 worin
X⁻ ein Anion ausgewählt aus der Gruppe Chlor, Brom, Iod, Methansulfonat oder Trifluormethansulfonat;
R Hydroxy, Methyl, CF₃ oder Fluor
R¹ und R² gleich oder verschieden, Methyl oder Ethyl, bevorzugt Methyl
R³, R⁴, R^{3'} und R^{4'}, gleich oder verschieden, Wasserstoff, -CF₃, -CHF₂ oder Fluor, bevorzugt Wasserstoff oder Fluor bedeuten,
   worin R³, R^{3'}, R⁴ und R^{4'} gleich oder verschieden, Wasserstoff, Methyl, Methoxy, Hydroxy, -CF₃, -CHF₂ oder Halogen und
R⁶ und R^{6'} jeweils Wasserstoff oder gemeinsam eine Einfachbindung; bedeuten.
dadurch gekennzeichnet, daß eine Verbindung der Formel 2, 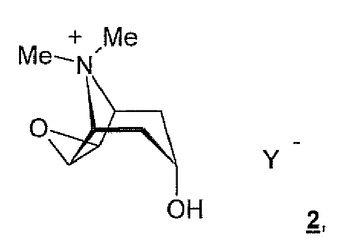 worin
Y⁻ Chlor, Brom, Iod, Methansulfonat oder Trifluormethansulfonat bedeuten kann und
R¹ und R² die oben genannten Bedeutungen haben kann,
in einem Schritt mit einer Verbindung der Formel 3 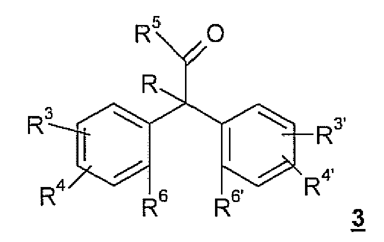 worin
- R⁵: ein Rest ausgewählt aus der Gruppe bestehend aus Hydroxy, Methoxy, Ethoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet undR⁶ und R^{6'} jeweils Wasserstoff oder gemeinsam eine Einfachbindung und
worin R, R³, R^{3'}, R⁴ und R^{4'} eine der vorstehenden Bedeutungen haben können, umgesetzt wird.

Besonders bevorzugt betrifft die vorliegende Erfindung ein Verfahren zur Herstellung von Verbindungen der Formel 1 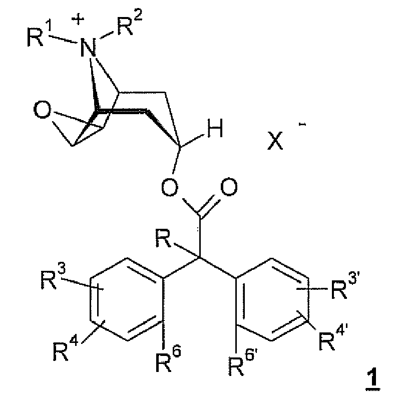 worin
- X⁻: Brom, Methansulfonat oder Trifluormethansulfonat;
- R: Hydroxy oder Methyl
- R¹ und R²: gleich oder verschieden, Methyl oder Ethyl, bevorzugt Methyl
- R3, R^{3'}, R⁴: und R^{4'} gleich oder verschieden, Wasserstoff, Methyl, Methoxy, Hydroxy, -CF₃, oder Fluor, bevorzugt Wasserstoff oder Fluor,
und
- R⁶ und R^{6'}: jeweils Wasserstoff oder gemeinsam eine Einfachbindung bedeuten,
dadurch gekennzeichnet, daß eine Verbindung der Formel **2**, 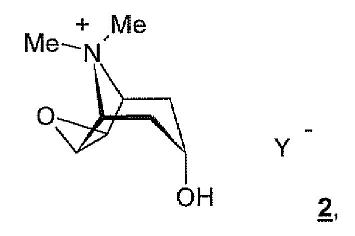 worin
- Y -: Brom, Methansulfonat oder Trifluormethansulfonat bedeuten kann und
- R¹ und R²: die oben genannten Bedeutungen haben kann,
in einem Schritt mit einer Verbindung der Formel **3** 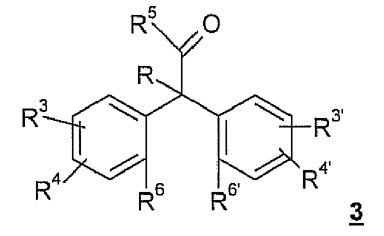 worin
- R⁵: ein Rest ausgewählt aus der Gruppe bestehend aus Hydroxy, O-N-Succinimid, O-N-Phtalimid, Vinyloxy und 2-Allyloxy, bevorzugt Vinyloxy und 2-Allyloxy, bedeutet und
die Reste R, R³, R⁴, R^{3'}, R^{4'}, R⁶ und R^{6'} eine der vorstehend genannten Bedeutungen haben können, umgesetzt wird.

In einer besonders bevorzugten Ausführungsform betrifft die vorliegende Erfindung ein Verfahren zur Herstellung von Verbindungen der Formel **1** 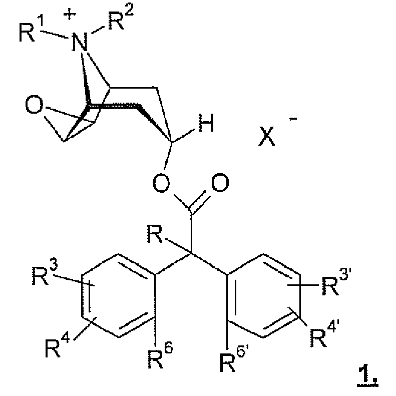 worin
X⁻ ein einfach negativ geladenes Anion, vorzugsweise Chlor, Brom, Iod, Methansulfonat oder Trifluormethansulfonat, insbesondere Brom;
R Methyl,
R¹ und R² jeweils Methyl
R³, R^{3'}, R⁴ und R^{4'} jeweils Wasserstoff und
R⁶ und R^{6'} gemeinsam eine Einfachbindung bedeuten,
dadurch gekennzeichnet, daß eine Verbindung der Formel 2 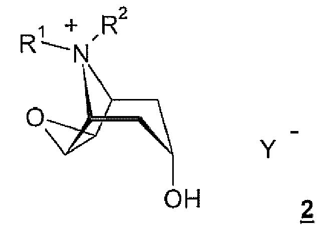 worin
Y⁻ ein einfach negativ geladenes Anion, vorzugsweise Chlor, Brom, Iod, Methansulfonat oder Trifluormethansulfonat, insbesondere Brom;
   und
R¹ und R² die oben genannten Bedeutungen haben kann,

in einem Schritt mit einer Verbindung der Formel **3** 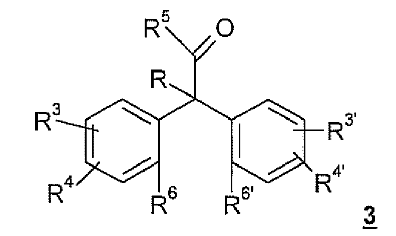 worin
- R⁵: ein Rest ausgewählt aus der Gruppe bestehend aus Hydroxy, Methoxy, Ethoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet und
die Reste R, R³, R⁴, R^{3'}, R^{4'}, R⁶ und R^{6'} eine der vorstehend genannten Bedeutungen haben, umgesetzt wird.

In einer besonders bevorzugten Ausführungsform betrifft die vorliegende Erfindung ein Verfahren zur Herstellung von Verbindungen der Formel **1** 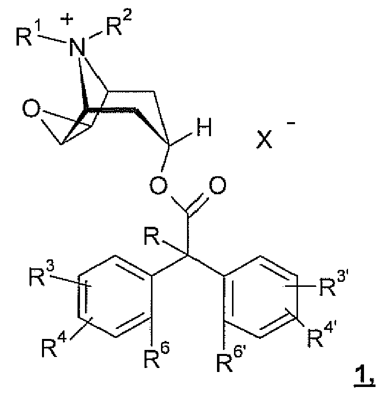 worin
- X -: ein einfach negativ geladenes Anion, vorzugsweise Chlor, Brom, Jod, Methansulfonat oder Trifluormethansulfonat, insbesondere Brom;
- R: Methyl,
- R¹ und R²: jeweils Methyl und
- R³, R⁴, R^{3'} und R^{4'}: jeweils Wasserstoff und
- R⁶ und R^{6'} jeweils: Wasserstoff
bedeuten,

dadurch gekennzeichnet, daß eine Verbindung der Formel **2** 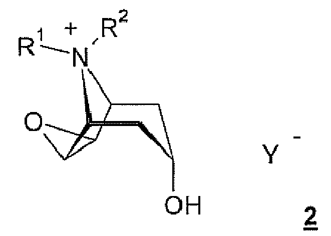 worin
- Y -: ein einfach negativ geladenes Anion, vorzugsweise Chlor, Brom, Iod, Methansulfonat oder Trifluormethansulfonat, insbesondere Brom; und
- R¹ und R²: jeweils Methyl bedeuten,

in einem Schritt mit einer Verbindung der Formel **3** 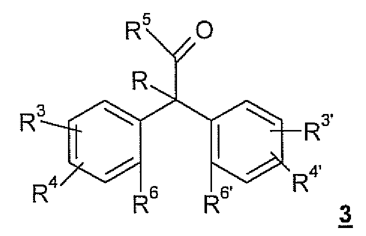 worin
R⁵ ein Rest ausgewählt aus der Gruppe bestehend aus Hydroxy, Methoxy, Ethoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet und
R Methyl und
R³, R⁴, R^{3'} und R^{4'} jeweils Wasserstoff und
R⁶ und R^{6'} jeweils Wasserstoff
bedeuten,
umgesetzt wird.

Zur Durchführung des erfindungsgemäßen Verfahrens kann wie nachfolgend beschrieben vorgegangen werden.
In einem ersten Schritt wird die Verbindung der Formel 3 in einem geeigneten organischen Lösmittel, vorzugsweise in einem polaren organischen Lösemittel, besonders bevorzugt in einem Lösemittel ausgewählt aus der Gruppe bestehend aus Acetonitril, Nitromethan, Formamid, Dimethylformamid, N-Methylpyrrolidinon, Dimethylsulfoxid und Dimethylacetamid aufgenommen, wobei von vorstehend genannten Lösemitteln Dimethylformamid, N-Methylpyrrolidinon und Dimethylacetamid besonders bevorzugt sind. Erfindungsgemäß von besonderer Bedeutung sind Dimethylformamid und N-Methylpyrrolidinon, wobei letzteres besonders bevorzugt ist.

Pro Mol engesetzte Verbindung der Formel **3** gelangen vorzugsweise zwischen 0,5 und 2 L, besonders bevorzugt zwischen 0,75 und 1,5 L des genannten Lösemittels zum Einsatz.

Je nach Wahl der Verbindung der Formel **3** kann es gegebenenfalls sinnvoll sein, diese vor Umsetzung mit der Verbindung der Formel **2** zu aktivieren. Werden als Verbindung der Formel 3 solche Derivate eingesetzt, in denen R = Hydroxyl bedeutet, ist beispielsweise der Einsatz entsprechender Aktivierungsreagenzien wie Carbonyldiimidazol, Carbonyldi-1,2,4-triazol, Dicyclohexylcarbodümid oder Ethyldimethylaminopropylcarbodiimid erfindungsgemäß bevorzugt, wobei in diesem Zusammenhang die Verwendung von Carbonyldiimidazol besonders bevorzugt ist. Pro Mol eingesetzte Verbindung **3** mit R=Hydroxy werden zwischen 1 - 2 Mol des Kopplungsreagenzes verwendet. Bevorzugt gelangen 1 - 1,5 Mol des Kopplungsreagenzes zum Einsatz. Gelangen vorstehend genannte Kopplungsreagentien, wie im Falle von R= Hydroxy bevorzugt, zum Einsatz wird die dann erhaltene Reaktionsmischung vorzugsweise in einem Temperaturbereich von 15-35°C, vorzugsweise bwi 20-25°C über einen Zeitraum von 1-8 Stunden, vorzugsweise 3-7 Stunden gerührt, bevor wie nachfolgend beschrieben weiter umgesetzt wird.

Die Reaktionsmischung von **3** in vorstehend genanntem Lösemittel, gegebenenfalls nach Zusatz eines der vorstehend genannten Kopplungsreagenzien im Falle von R=Hydroxy, wird sodann auf eine Temperatur von kleiner 30°C, vorzugsweise auf eine Temperatur zwischen -20°C und 20°C, besonders bevorzugt auf eine Temperatur zwischen -10°C und 5°C eingestellt und mit der Verbindung der Formel **2** versetzt. Bezogen auf ursprünglich eingesetzte Verbindung **3** können stöchiometrische Mengen an Verbindung der Formel **2** zugegeben werden. Erfindungsgemäß bevorzugt liegt **3** allerdings im Vergleich zu **2** im Überschuß vor. Pro Mol eingesetzte Verbindung **3** gelangen erfindungsgemäß zwischen 0,5 und 1 Mol, vorzugsweise zwischen 0,7 und 0,95 Mol, besonders bevorzugt zwischen 0,75 und 0,9 Mol **2** zur Anwendung.

Vorstehend genannte Reaktionsmischung wird anschließend mit einer in einem der vorstehend genannten Lösemittel gelösten geeigneten Base versetzt. Hierbei können organische oder anorganische Basen zum Einsatz gelangen. Als organische Basen werden bevorzugt Alkaliimidazolide , welche besiepielsweise in situ aus den Alkalimetallen und Imidazol oder den Alkalimetallhydriden und Imidazol generiert werden können, oder Alkalimetall-tertiär-Amylate, die kommerziell erhältlich sind, verwendet. Als Akaliimidazolide kommen bevorzugt Imidazolide des Lithiums, Natriums oder Kaliums in Betracht, wobei Natrium- oder Lithiumimidazolid erfindungsgemäß bevorzugt sind. Besonders bevorzugt gelangt Lithiumimidazolid zum Einsatz. Als Alkalimetall-tertiär-Amylate kommen vorzugsweise Natrium-tertiär-Amylat und Kalium-tertiär-Amylat, insbesondere aber Natrium-tertiär-Amylat zum Einsatz. Als anorganische Base kommen vorzugsweise Hydride des Lithiums, Natriums oder Kaliums in Betracht. Besonders bevorzugt wird als anorganische Base Natriumhydrid eingesetzt. Von allen vorstehend genannten Basen gelangt besonders bevorzugt Lithiumimidazolid zur Anwendung.

Sollen Verbindungen der Formel **1** erhalten werden, in denen R für Hydroxy steht steht, kann anstelle der vorstehend genannten basenkatalysierten Umsetzung auch eine Umesterung unter milderen Reaktionsbedingungen vorteilhaft erscheinen. Hierbei können vorteilhaft Zeolithe als Katalysatoren genutzt werden.

Wird die Umsetzung mit einer der vorstehend genannten Basen durchgeführt, werden pro Mol eingesetzte Verbindung **2** wenigstens stöchiometrische Mengen an Base eingesetzt. Bevorzugt werden pro Mol eingesetzte Verbindung **2** 1 bis 1,5 Mol, vorzugsweise 1,1 bis 1,3 Mol Base verwendet. Wird die Base in Form einer Lösung zugegeben, was insbesondere im Falle der erfindungsgemäß bevorzugten und zuvor in situ generierten Base Lithiumimidazolid der Fall ist, gelangt hierfür vorzugsweise dasjenige Lösemittel zur Anwendung, welches bereits zur Durchführung der vorstehend genannten Schritte Verwendung findet. Pro Mol engesetzte Base gelangen vorzugsweise zwischen 0,3 und 1,3 L, besonders bevorzugt zwischen 0,5 und 1 L des genannten Lösemittels zum Einsatz. Nach beendeter Basenzugabe wird in einem Temperaturbereich von 15-35°C, vorzugsweise bwi 20-25°C über einen Zeitraum von 4-48 Stunden, vorzugsweise 8-36 Stunden gerührt.

Zu der so entstandenen Suspension wird bei konstanter Temperatur eine Säure H-X gegeben. Die Wahl der Säure bestimmt sich dabei nach dem Anion X- im gewünschten Endprodukt der allgemeinen Formel **1**. Insofern im Rahmen der vorliegenden Erfindung bevorzugt solche Verbindungen der allgemeinen Formel **1** synthetisiert werden, in denen X- für Bromid steht, wird die nachfolgende Vorgehensweise für die Herstellung der erfindungsgemäß bevorzugten Bromidhaltigen Endprodukte der Formel **1** beschrieben. Für den Fachmann ist ersichtlich, daß eine entsprechende Vorgehensweise durch Wahl des geeigneten Reagenzes H-X in analoger Art und Weise auch zur Herstellung solcher Verbindungen Verwendung finden kann, in denen X- nicht Bromid bedeutet.

Zur Herstellung von Verbindungen der Formel **1** mit X = Bromid werden bezogen auf eingesetzte Verbindung der Formel **3** vorzugsweise 2 bis 4 Mol, bevorzugt 2 bis 3 Mol, besonders bevorzugt 2,2 bis 2,6 Mol Bromwasserstoff bei konstanter Temperatur gegeben. Der verwendete Bromwasserstoff kann hierbei entweder in gasförmiger Form oder in Form, vorzugsweise gesättigter, Lösungen zugegeben werden. Erfindungsgemäß bevorzugt erfolgt die Zugabe an Bromwasserstoff in Eisessig gelöster Form. Besonders bevorzugt gelangt hierbei eine 33%-ige Bromwasserstoff-Lösung in Eisessig zum Einsatz. Nach beendeter Zugabe wird bei konstanter Temperatur, gegebenfalls auch unter Eiskühlung nachgerührt (zwischen 0,5 und 6 Stunden)

Abschließend wird die erhaltene Lösung mit einem unpolareren organischen Lösemittel, vorzugsweise mit einem Lösmittel ausgewählt aus der Gruppe bestehend aus Aceton, Toluol, n-Buthylacetat, Dichlormethan, Diethylether, Tetrahydrofuran und Dioxan, besonders bevorzugt Toluol oder Aceton versetzt.

Nach guter Durchmischung wird das auskristallisierte Produkt abgetrennt und mit dem vorstehend genannten unpolaren Lösemittel gewaschen. Zur Abtrennung wasserlöslicher Verunreinigungen kann das Rohprodukt mit wässerigen Bromidlösungen z.B. Natrium oder Kaliumbromidlösung behandelt werden.

Eine weitergehende Reinigung der so erhaltenen Verbindungen der Formel **1** kann, sofern erforderlich, durch Chromatographie über Kieselgel oder mittels Umkristallisation aus geeigneten Lösemitteln wie z.B. Wasser oder niederen Alkoholen, wie beispielsweise Isopropanol erfolgen.

Durch die Verwendung der im Stand der Technik bekannten Verbindungen der Formel **2** als Ausgangsmaterialien zur Synthese der Strukturen der Formel **1** gelingt ein Zugang zu diesen anticholinerg wirksamen Strukturen in nur einem Reaktionsschritt.
Dementsprechend betrifft ein weiterer Aspekt der vorliegenden Erfindung die Verwendung von Verbindungen der Formel **2** 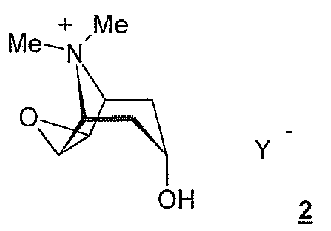 worin
- Y -: Chlor, Brom, Iod, Methansulfonat oder Trifluormethansulfonat bedeuten
als Ausgangsmaterial zur Herstellung von Verbindungen der Formel **1** 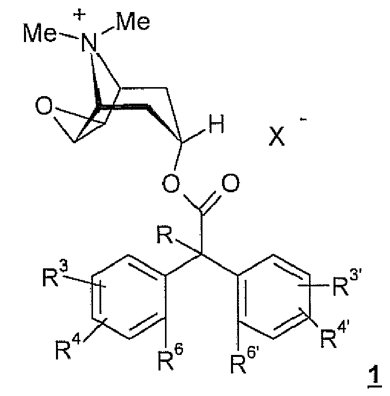 worin
X⁻ Chlor, Brom, Iod, Methansulfonat oder Trifluormethansulfonat;
R Hydroxy, C₁-C₄-Alkyl, C₁-C₄-Alkoxy, CF₃ oder Fluor und
R³, R⁴, R³' und R^{4'}, gleich oder verschieden, Wasserstoff, -C₁-C₄-Alkyl, -C₁-C₄-Alkyloxy, Hydroxy, -CF₃, -CHF₂, CN, NO₂ oder Halogen und
R⁶ und R^{6'} jeweils Wasserstoff oder gemeinsam eine Einzelbindung bedeuten.

Bevorzugt betrifft die vorliegende Erfindung die Verwendung von Verbindungen der Formel **2** 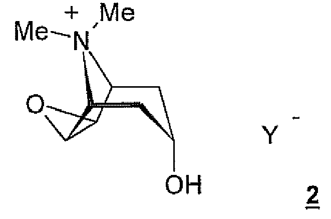 worin
- Y -: Brom, Methansulfonat oder Trifluormethansulfonat bedeuten
als Ausgangsmaterial zur Herstellung von Verbindungen der Formel **1** 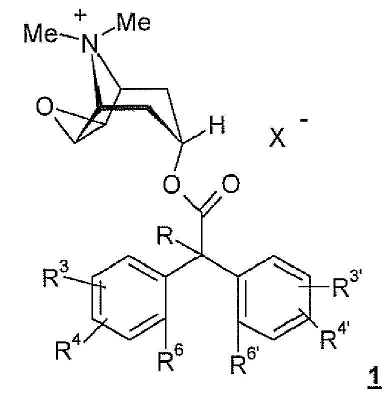 worin
X⁻ Brom, Iod, Methansulfonat oder Trifluormethansulfonat;
R Hydroxy, Methyl, CF₃ oder Fluor und
R3, R⁴, R^{3'} und R^{4'}, gleich oder verschieden, Wasserstoff, Methyl, Methoxy, Hydroxy, -CF₃, -CHF₂ oder Halogen und
R⁶ und R^{6'} jeweils Wasserstoff oder gemeinsam eine Einzelbindung bedeuten.

Besonders bevorzugt betrifft die vorliegende Erfindung die Verwendung von Verbindungen der Formel **2** 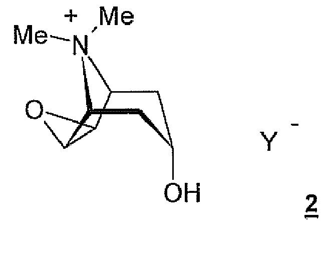 worin
- Y -: Brom, Methansulfonat oder Trifluormethansulfonat bedeuten
als Ausgangsmaterial zur Herstellung von Verbindungen der Formel **1** 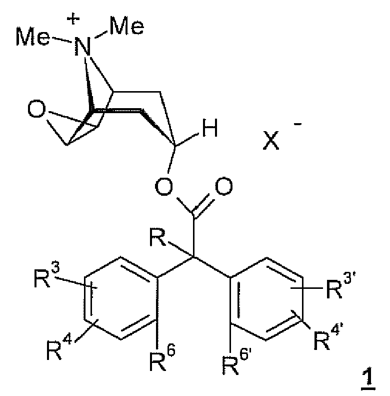 worin
X⁻ Brom, Methansulfonat oder Trifluormethansulfonat;
R Hydroxy oder Methyl;
R³, R⁴, R³' und R^{4'}, gleich oder verschieden, Wasserstoff, Methyl, Methoxy, Hydroxy, -CF₃ oder Fluor und
R⁶ und R^{6'} jeweils Wasserstoff oder gemeinsam eine Einzelbindung
bedeuten.

In einer besonders bevorzugten Ausführungsform betrifft die Erfindung die Verwendung einer Verbindung der Formel **2** 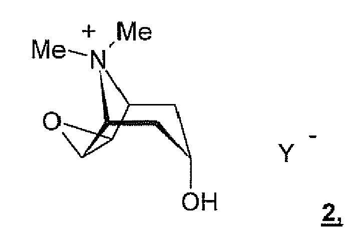 worin
- Y -: Chlor, Brom, Iod, Methansulfonat oder Trifluormethansulfonat bedeutet,
als Ausgangsmaterial zur Herstellung von Verbindungen der Formel **1** 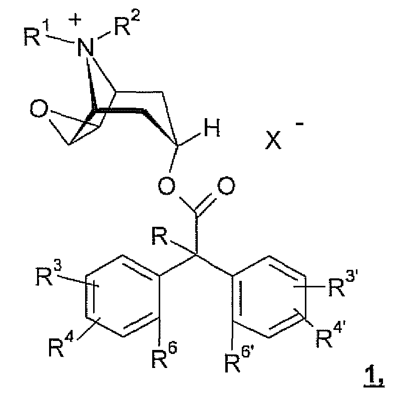 worin
X⁻ Chlor, Brom, Iod, Methansulfonat oder Trifluormethansulfonat;
R Hydroxy, C₁-C₄-Alkyl, C₁-C₄-Alkoxy, CF₃ oder Fluor; vorzugsweise Methyl
R3, R⁴, R^{3'} und R^{4'}, gleich oder verschieden, Wasserstoff, -C₁-C₄-Alkyl, -C₁-C₄-Alkyloxy, Hydroxy, -CF₃, -CHF₂, CN, NO₂ oder Halogen, vorzugsweise jeweils Wasserstoff, und
R⁶ und R^{6'} gemeinsam eine Einfachbindung,
bedeuten,
vorzugsweise in Gegenwart eines Alkalimetallsalzes von tertiär-Amylat als Base, insbesondere in Gegenwart von Natrium-tertiär-Amylat als Base.

Die nachfolgenden Beispiele dienen der Illustration exemplarisch durchgeführter Syntheseverfahren. Sie sind lediglich als mögliche, exemplarisch dargestellte Vorgehensweisen zu verstehen, ohne die Erfindung auf deren Inhalt zu beschränken.

In einer besonders bevorzugten Ausführungsform betrifft die vorliegende Erfindung die Verwendung von Verbindungen der Formel **2** 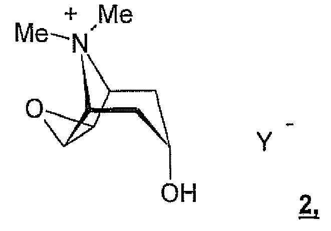 worin
- Y -: Brom bedeutet,
als Ausgangsmaterial zur Herstellung von Verbindungen der Formel **1**, 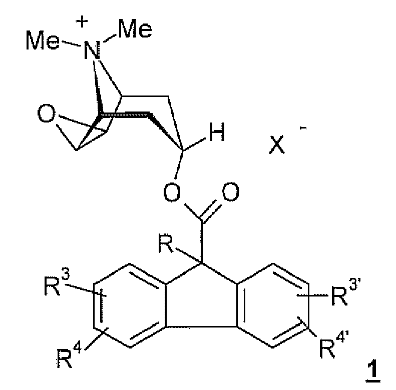 worin
- X -: Brom,
- R: Methyl und
- R³, R⁴, R³' und R⁴': Wasserstoff bedeuten.

### Beispiel 1 : 9-Methyl-fluoren-9-carbonsäurescopinester-Methobromid

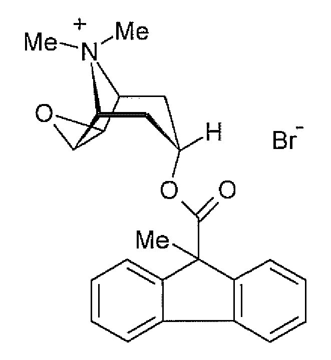

Zu einer Lösung bestehend aus 710,8 g (4,384 Mol) 1,1-Carbonyldiimidazol in 8,43 Liter N-Methylpyrrolidon gibt man 900,0 g (4,013 Mol) 9-Methyl-fluoren-9-carbonsäure und rührt die Reaktionsmischung 2,5-3,0 Stunden bei Raumtemperatur. Anschließend werden 843,4 g (3,372 Mol) Scopinmethobromid sowie 408,4 g (3,709 Mol) Natrium-tert.-amylat portionsweise zugegeben. Das Reaktionsgemisch wird für 3,0-3,5 Stunden auf 40-45°C erwärmt. Anschließend wird der Reaktorinhalt auf 0-3°C abgekühlt und bei einer Temperatur von 0-7°C 1,38 Liter HBr, 33%ig in Eisessig, langsam zugegeben. Nach Ende der Zugabe wird für 15 Minuten nachgerührt und anschließend 9,64 Liter Isopropanol zudosiert. Die Suspension wird über Nacht bei 0-3°C nachgerührt, filtriert, der Filterkuchen mit 1,5 Liter Isopropanol nachgewaschen und anschl. im Vakuum bei 45°C getrocknet. Das so erhaltene Rohprodukt wird anschließend in 7,39 Liter G-Wasser bei 60°C gelöst. Durch Zugabe von Bromwasserstoffsäure 48%ig wird der pH Wert der Lösung auf einen Wert im Bereich pH 3,0 bis 4,0 eingestellt. Die Lösung wird über Aktiv-Kohle klarfiltriert, das Filtrat auf 0-3°C abgekühlt wobei das Produkt kristallisiert. Die so erhaltene Suspension wird 4-5 Stunden bei 0-3°C nachgerührt, abgesaugt, mit 1,5 Liter Wasser nachgewaschen und im Vakuumtrockenschrank bei 45°C getrocknet.

In analoger Art und Weise kann in einem Syntheseschritt erhalten werden:

### Beispiel 2 : 9-Fluor-fluoren-9-carbonsäurescopinester Methobromid

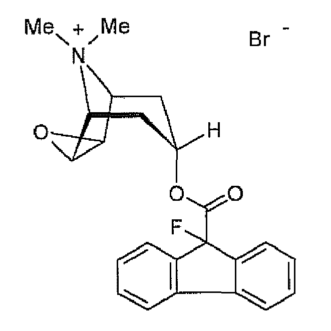

### Beispiel 3: 2,2-Diphenylpropionsäurescopinester-Methobromid 1. Herstellung von : 2,2-Diphenylpropionsäurescopinester-Methobromid (Rohprodukt):

10,53 kg (64,97 mol) 1,1-Carbonyldiimidazol werden in 101,8 L N-Methylpyrrolidon gelöst. Bei einer Produkttemperatur von 25°C werden portionsweise 14,70 kg (64,97 mol) 2,2-Diphenylpropionsäure eingetragen. Der Reaktorinhalt wird 2 Stunden bei 25°C gerührt und dann auf 2°C abgekühlt. 12,50 kg (49,79 mol) BEA 2180 Alkohol und 6,54 kg (59,37 mol) Natrium tert.-amylat werden nacheinander bei maximal 7°C zugegeben. Der Trichter wird mit 8,9 L N-Methylpyrrolidon gespült. Die erhaltene Suspension wird 20 Minuten bei 7°C gerührt. Der Reaktorinhalt wird auf 25°C erwärmt und bei dieser Temperatur 5 Stunden gerührt. Anschließend werden 125 L Isopropylalkohol bei 16°C zugegeben und das Reaktionsgemisch bei 16 - 21 °C mit 28,59 kg (116,59 mol) Bromwasserstoff 33% in Essigsäure versetzt. Das Zulaufgefäß wird mit 17,9 L Isopropylalkohol gespült. Die Suspension wird bei 23°C 14 Std. gerührt. Der Reaktorinhalt wird innerhalb von 30 Minuten auf 2°C abgekühlt und 3 Stunden bei dieser Temperatur gerührt. Das Rohprodukt wird abzentrifugiert, zweimal mit je 35,7 L Isopropylalkohol gewaschen und im Vakuumtrockenschrank im Vakuum mit Stickstoff als Schleppgas bei 45°C getrocknet.

### 2. Umkristallisation aus Isopropanol:

8,00 kg (17,45 mol) des 2,2-Diphenylpropionsäurescopinester-Methobromid-Rohprodukteswerden in 168 L Isopropylalkohol unter Rückfluss gelöst. Nach Abkühlen knapp unter die Rückflusstemperatur (79°C) wird über einen Druckfilter in einen 2. Reaktor klarfiltriert. Über den 1. Reaktor wird der Druckfilter und die Schläuche mit 16 L Isopropylalkohol gespült. Das Filtrat im 2. Reaktor wird zum Rückfluß erhitzt. Nach Abkühlen auf 70°C wird mit 3,2 g bereits aus Isopropanol umkristallisiertem 2,2-Diphenylpropionsäurescopinester-Methobromid angeimpft und innerhalb von 3,5 Std. auf 0°C abgekühlt. Zur Vervollständigung der Kristallisation wird noch 3 Std. bei 0°C gerührt. Die Suspension wird abzentrifugiert und das Produkt mit 32 L kaltem Isopropylalkohol gewaschen. Das aus Isopropanol umkristallisierte 2,2-Diphenylpropionsäurescopinester-Methobromid wird bei 45°C im Vakuumtrockner getrocknet, dann im Reibschnitzler durch ein 2 mm Sieb passiert.

### 3. Umkristallisation aus Wasser:

10,00 kg (21,8 mol) 2,2-Diphenylpropiansäurescapinester-Methobromid werden in 70 L G-Wasser bei 40°C gelöst. Die Lösung wird auf 25°C abgekühlt und mit 5,0 g aus Wasser umkristallisiertem 2,2-Diphenylpropionsäurescopinester-Methobromid angeimpft. Innerhalb von 30 Minuten wird auf 20°C abgekühlt und 2 Std. bei dieser Temperatur gerührt. Innerhalb von 1 Std. wird auf 0°C abgekühlt und 2,5 Std. bei dieser Temperatur gerührt. Die Suspension wird abzentrifugiert und das Produkt mit 20 L eiskaltem G-Wasser gewaschen. Das aus Wasser umkristallisierte 2,2-Diphenylpropionsäurescopinester-Methobromid wird bei 50°C im Vakuumtrockenschrank getrocknet.

Je nach Reinheitsgrad des 2,2-Diphenylpropionsäurescopinester-Methobromid-Rohproduktes kann entweder nur aus Isopropanol oder aus Isopropanol und anschließend aus Wasser umkristallisiert werden.

## Patentansprüche

1. Verfahren zur Herstellung von Verbindungen der Formel **1** 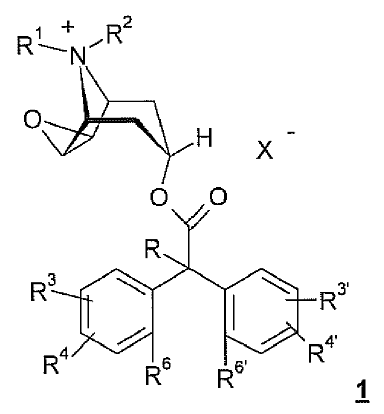 worin
X⁻ ein einfach negativ geladenes Anion,
R Wasserstoff, Hydroxy, Methyl, Ethyl, -CF₃, CHF₂ oder Fluor;
R¹ und R² gleich oder verschieden, -C₁-C₅-Alkyl, welches gegebenenfalls durch -C₃-C₆-Cycloalkyl, Hydroxy oder Halogen substituiert sein kann,
oder
R¹ und R² gemeinsam eine -C₃-C₅-Alkylen-Brücke;
R³, R⁴, R^{3'} und R^{4'}, gleich oder verschieden, Wasserstoff, -C₁-C₄-Alkyl, -C₁-C₄-Alkyloxy, Hydroxy, -CF₃, -CHF₂, CN, NO₂ oder Halogen
R⁶ und R^{6'} Wasserstoff oder gemeinsam eine Einfachbindung; bedeuten,
**dadurch gekennzeichnet, daß** eine Verbindung der Formel **2** 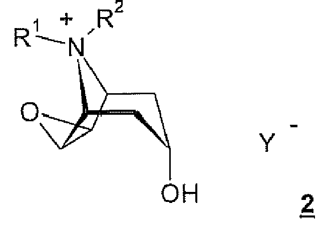 worin
Y - ein einfach negativ geladendes Anion, und
R¹ und R² die obigen Bedeutungen haben,
in einem Schritt mit einer Verbindung der Formel **3** 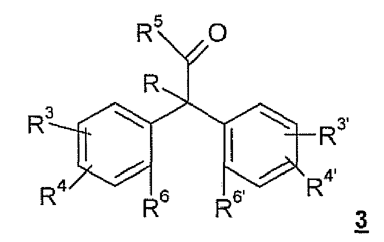 worin
R⁵ ein Rest ausgewählt aus der Gruppe bestehend aus Hydroxy, Methoxy, Ethoxy, O-N-Succinimid, O-N-Phtalimid, Phenyloxy, Nitrophenyloxy, Fluorophenyloxy, Pentafluorophenyloxy, Vinyloxy, 2-Allyloxy, -S-Methyl, -S-Ethyl und -S-Phenyl bedeutet und
worin R, R3, R^{3'}, R⁴, R^{4'}, R⁶ und R^{6'} die vorstehend genannten Bedeutungen haben, umgesetzt wird.

2. Verfahren nach Anspruch 1, zur Herstellung von Verbindungen der Formel **1**, worin
X⁻ ein Anion ausgewählt aus der Gruppe bestehend aus Chlor, Brom, Iod, Methansulfonat oder Trifluormethansulfonat
R Hydroxy, Methyl, CF₃ oder Fluor;
R¹ und R² gleich oder verschieden, Methyl oder Ethyl,
R³, R^{3'}, R⁴ und R^{4'} gleich oder verschieden, Wasserstoff, Methyl, Methoxy, Hydroxy, -CF₃, -CHF₂ oder Halogen und
R⁶ und R^{6'} Wasserstoff oder gemeinsam eine Einfachbindung, bedeuten,
**dadurch gekennzeichnet, daß** eine Verbindung der Formel **2**, worin
Y - Chlor, Brom, Iod, Methansulfonat oder Trifluormethansulfonat bedeuten kann, und
die Reste R¹ und R² eine der vorstehend genannten Bedeutungen haben können,
in einem Schritt mit einer Verbindung der Formel **3**, worin
R⁵ ein Rest ausgewählt aus der Gruppe bestehend aus Hydroxy, O-N-Succinimid, O-N-Phtalimid, Vinyloxy und 2-Allyloxy bedeutet,
R⁶ und R^{6'} Wasserstoff oder gemeinsam eine Einfachbindung,
und
die Reste R, R³, R⁴, R^{3'} und R^{4'} eine der vorstehend genannten Bedeutungen haben können,
umgesetzt wird.

3. Verfahren nach Anspruch 1 oder 2 zur Herstellung einer Verbindung der Formel **1** , worin
X⁻ Brom, Methansulfonat oder Trifluormethansulfonat;
R Hydroxy oder Methyl;
R¹ und R² gleich oder verschieden, Methyl oder Ethyl,
R3, R^{3'}, R⁴ und R^{4'} gleich oder verschieden, Wasserstoff, Methyl, Methoxy, Hydroxy, -CF₃ und Fluor, und
R⁶ und R^{6'} Wasserstoff oder gemeinsam eine Einfachbindung,
bedeuten,
**dadurch gekennzeichnet, daß** eine Verbindung der Formel **2,** worin
Y⁻ Brom, Methansulfonat oder Trifluormethansulfonat bedeuten kann, und
die Reste R¹ und R² eine der vorstehend genannten Bedeutungen haben können,
in einem Schritt mit einer Verbindung der Formel **3**, worin
R⁵ ein Rest ausgewählt aus der Gruppe bestehend aus Hydroxy, O-N-Succinimid, O-N-Phtalimid, Vinyloxy und 2-Allyloxy, bevorzugt Vinyloxy und 2-Allyloxy,
R⁶ und R^{6'} Wasserstoff oder gemeinsam eine Einfachbindung, und die Reste R, R³, R⁴, R^{3'} und R^{4'} eine der vorstehend genannten Bedeutungen haben können,
umgesetzt wird.

4. Verfahren nach einem der Ansprüche 1 bis 3 zur Herstellung einer Verbindung der Formel **1** , worin
X⁻ Brom
R Methyl;
R¹ und R² jeweils Methyl
R3, R^{3'}, R⁴ und R^{4'} jeweils Wasserstoff und
R⁶ und R^{6'} gemeinsam eine Einfachbindung bedeuten,
**dadurch gekennzeichnet, daß** eine Verbindung der Formel 2, worin
Y⁻ Brom und
R¹ und R² jeweils Methyl bedeuten
in einem Schritt mit einer Verbindung der Formel **3**, worin
R⁵ ein Rest ausgewählt aus der Gruppe bestehend aus Hydroxy, O-N-Succinimid, O-N-Phtalimid, Vinyloxy und 2-Allyloxy, bevorzugt Vinyloxy und 2-Allyloxy,
R Methyl und
R³, R⁴, R^{3'} und R^{4'} jeweils Wasserstoff und
R⁶ und R^{6'} gemeinsam eine Einfachbindung bedeuten,
umgesetzt wird.

5. Verfahren nach einem der Ansprüche 1 bis 3 zur Herstellung einer Verbindung der Formel **1** , worin
X⁻ Brom
R Methyl;
R¹ und R² jeweils Methyl
R³, R^{3'}, R⁴ und R^{4'} jeweils Wasserstoff und
R⁶ und R^{6'} jeweils Wasserstoff bedeuten,
**dadurch gekennzeichnet, daß** eine Verbindung der Formel 2, worin
Y⁻ Brom und
R¹ und R² jeweils Methyl bedeuten
in einem Schritt mit einer Verbindung der Formel 3, worin
R⁵ ein Rest ausgewählt aus der Gruppe bestehend aus Hydroxy, O-N-Succinimid, O-N-Phtalimid, Vinyloxy und 2-Allyloxy, bevorzugt Vinyloxy und 2-Allyloxy,
R Methyl und
R³, R⁴, R^{3'} und R^{4'} jeweils Wasserstoff und
R⁶ und R^{6'} jeweils Wasserstoff bedeuten,
umgesetzt wird.

6. Verfahren nach einem der Ansprüche 1 bis 5, **dadurch gekennzeichnet, daß** die Umsetzung in einem organischen Lösemittel ausgewählt aus der Gruppe bestehend aus Acetonitril, Nitromethan, Formamid, Dimethylformamid, N-Methylpyrrolidinon, Dimethylsulfoxid und Dimethylacetamid durchgeführt wird.

7. Verfahren nach einem der Ansprüche 1 bis 6, **dadurch gekennzeichnet, daß** im Falle der Verwendung einer Verbindung der Formel **3**, in der R⁵ =OH bedeutet, Aktivierungsreagenzien ausgewählt aus der Gruppe Carbonyldiimidazol, Carbonyldi-1,2,4-triazol, Dicyclohexylcarbodiimid und Ethyldimethylaminopropylcarbodiimid zur Anwendung gelangen.

8. Verfahren nach einem der Ansprüche 1 bis 7, **dadurch gekennzeichnet, daß** die Umsetzung bei einer Temperatur von kleiner 30°C, vorzugsweise auf eine Temperatur zwischen -20°C und 20°C durchgeführt wird.

9. Verfahren nach einem der Ansprüche 1 bis 8, **dadurch gekennzeichnet, daß** die Umsetzung in Gegenwart einer organischen oder anorganischen Base durchgeführt wird.

10. Verfahren nach Anspruch 9, **dadurch gekennzeichnet, daß** die Umsetzung in Gegenwart eines Alkalimetallsalzes von tertiär-Amylat als Base durchgeführt wird.

11. Verfahren nach Anspruch 10, **dadurch gekennzeichnet, daß** die Umsetzung in Gegenwart von Natrium-tertiär-Amylat als Base durchgeführt wird.

12. Verfahren nach einem der Ansprüche 1 bis 11, **dadurch gekennzeichnet, daß** in dem Fall, in dem in den Verbindungen der Formel **1** R für Hydroxy steht, die Umsetzung in Gegenwart von Zeolithe als Katalysator durchgeführt wird.

13. Verwendung einer Verbindung der Formel **2** 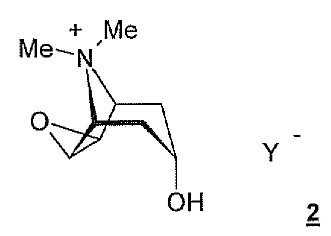 worin
Y - Chlor, Brom, Iod, Methansulfonat oder Trifluormethansulfonat bedeutet,
als Ausgangsmaterial zur Herstellung von Verbindungen der Formel **1** 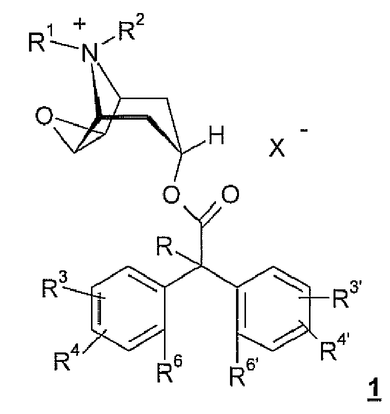 worin
X⁻ Chlor, Brom, Iod, Methansulfonat oder Trifluormethansulfonat;
R Hydroxy, C₁-C₄-Alkyl, C₁-C₄-Alkoxy, CF₃ oder Fluor;
R³, R⁴, R^{3'} und R^{4'}, gleich oder verschieden, Wasserstoff, -C₁-C₄-Alkyl, -C₁-C₄-Alkyloxy, Hydroxy, -CF₃, -CHF₂, CN, NO₂ oder Halogen, und R⁶ und R^{6'} gemeinsam eine Einfachbindung bedeuten.

14. Verwendung einer Verbindung der Formel **2** 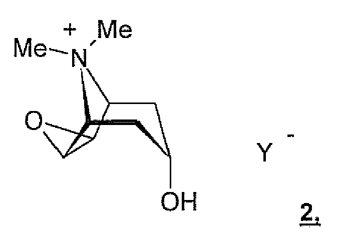 worin
Y - Chlor, Brom, Iod, Methansulfonat oder Trifluormethansulfonat bedeutet,
als Ausgangsmaterial zur Herstellung von Verbindungen der Formel **1** 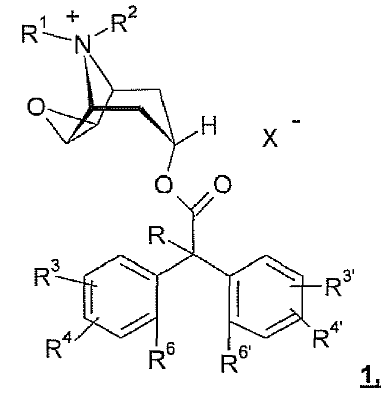 worin
X⁻ Chlor, Brom, Iod, Methansulfonat oder Trifluormethansulfonat;
R Hydroxy, C₁-C₄-Alkyl, C₁-C₄-Alkoxy, CF₃ oder Fluor;
R³, R⁴, R^{3'} und R^{4'}, gleich oder verschieden, Wasserstoff, -C₁-C₄-Alkyl, -C₁-C₄-Alkyloxy, Hydroxy, -CF₃, -CHF₂, CN, NO₂ oder Halogen, und
R⁶ und R^{6'} gemeinsam eine Einfachbindung,
bedeuten,
in Gegenwart eines Alkalimetallsalzes von tertiär-Amylat als Base, insbesondere in Gegenwart von Natrium-tertiär-Amylat als Base.
